(19) Europäisches Patentamt / European Patent Office / Office européen des brevets

(11) **EP 3 868 296 A1**

(12) **EUROPEAN PATENT APPLICATION**
published in accordance with Art. 153(4) EPC

(43) Date of publication:
**25.08.2021 Bulletin 2021/34**

(51) Int Cl.:
**A61B 5/1455** (2006.01)   **G01N 21/17** (2006.01)

(21) Application number: **19874504.4**

(22) Date of filing: **16.10.2019**

(86) International application number:
**PCT/JP2019/040692**

(87) International publication number:
**WO 2020/080409 (23.04.2020 Gazette 2020/17)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.10.2018 JP 2018195906**

(71) Applicant: **Medical Photonics Co., Ltd.**
**Sapporo-shi, Hokkaido 001-0021 (JP)**

(72) Inventor: **IINAGA, Kazuya**
**Sapporo-shi, Hokkaido 001-0021 (JP)**

(74) Representative: **Vossius & Partner**
**Patentanwälte Rechtsanwälte mbB**
**Siebertstraße 3**
**81675 München (DE)**

(54) **PARTICLE CONCENTRATION MEASUREMENT DEVICE, PARTICLE CONCENTRATION MEASUREMENT PROGRAM, AND PARTICLE CONCENTRATION MEASUREMENT METHOD**

(57) This particle concentration measurement device is used under time-periodic illumination light and has: a received light intensity detection unit for detecting the intensity of received light emitted from a subject; and a control unit for determining the amplitude from the intensity of received light and calculating the concentration of particles within the subject from the amplitude.

FIG. 1

(a)

(b)

EP 3 868 296 A1

**Description**

Technical Field

An embodiment described in the present specification relates to a particle concentration measurement device, a particle concentration measurement program, and a particle concentration measurement method capable of reducing a measurement error due to a measurement site.

Background Art

In noninvasive in-blood lipid measurement of related art, a scattering coefficient is determined from the degree of attenuation of light attenuated as the result of an increase in light reception distance, and the scattering coefficient is converted into lipid concentration (see Patent Literature 1, for example).

Citation List

Patent Literature

Patent Literature 1: International Publication No. 2014/087825

Summary of Invention

Technical Problem

In the related art, however, it is necessary to perform the measurement in a position where the uniformity of particle concentration is relatively high, and it is therefore cumbersome to search for an optimum measurement position.
An object of the present invention is to reduce the effort of detection of the optimum measurement site.

Solution to Problem

A particle concentration measurement device according to the present invention is a particle concentration measurement device used under temporally periodic illumination light, the particle concentration measurement device including a received light intensity detection unit that detects an intensity of light emitted from a subject and received by the received light intensity detection unit and a control unit that determines an amplitude from the received light intensity and calculates a concentration of particles in the subject from the amplitude.
A particle concentration measurement device according to the present invention includes a radiator that radiates temporally periodic light to a subject, a received light intensity detection unit that is so disposed as to be separate from the radiator by a predetermined distance and detects an intensity of light emitted from the subject and received by the received light intensity detection unit, and a control unit that determines an amplitude from the received light intensity and calculates a concentration of particles in the subject from the amplitude.
A particle concentration measurement device according to the present invention is a particle concentration measurement device communicably connected to a user device used under temporally periodic illumination light and including a received light intensity detection unit that detects an intensity of light emitted from a subject and received by the received light intensity detection unit, the particle concentration measurement device including a control unit that determines an amplitude from the received light intensity transmitted from the user device and calculates a concentration of particles in the subject from the amplitude.
A particle concentration measurement device according to the present invention is a particle concentration measurement device communicably connected to a user device including a radiator that radiates temporally periodic light to a subject and a received light intensity detection unit that is so disposed as to be separate from the radiator by a predetermined distance and detects an intensity of light emitted from the subject and received by the received light intensity detection unit, the particle concentration measurement device including a control unit that determines an amplitude from the received light intensity transmitted from the user device and calculates a concentration of particles in the subject from the amplitude.
A particle concentration measurement method according to the present invention is a particle concentration measurement method performed under temporally periodic illumination light, the method including detecting an intensity of light emitted and received from a subject, determining an amplitude from the received light intensity, and calculating a concentration of particles in the subject from the amplitude.
A particle concentration measurement method according to the present invention includes radiating temporally periodic light to a subject, detecting an intensity of light emitted and received from the subject and so disposed as to separate from the radiator by a predetermined distance, determining an amplitude from the received light intensity, and calculating a concentration of particles in the subject from the amplitude.

A particle concentration measurement program according to the present invention causes a computer of a device communicably connected to a user device used under temporally periodic illumination light and including a received light intensity detection unit that detects an intensity of light emitted from a subject and received by the received light intensity detection unit to carry out the process of determining an amplitude from the received light intensity transmitted from the user device and calculating a concentration of particles in the subject from the amplitude.

A particle concentration measurement program according to the present invention causes a computer of a device communicably connected to a user device including a radiator that radiates temporally periodic light to a subject and a received light intensity detection unit that is so disposed as to be separate from the radiator by a predetermined distance and detects an intensity of light emitted from the subject and received by the received light intensity detection unit to carry out the process of determining an amplitude from the received light intensity transmitted from the user device and calculating a concentration of particles in the subject from the amplitude.

Brief Description of Drawings

[Figure 1] Figure 1 schematically shows a particle concentration measurement device.
[Figure 2] Figure 2 shows the configuration of a control system of the particle concentration measurement device.
[Figure 3] Figure 3 shows a simulation.
[Figure 4] Figure 4 shows the result of the simulation.
[Figure 5] Figure 5 shows the depth versus the luminance.
[Figure 6] Figure 6 shows the result of measurement of received light intensity in a skin layer.
[Figure 7] Figure 7 shows correlation between lipid concentration and light propagation efficiency.
[Figure 8] Figure 8 shows the correlation between the lipid concentration and the light propagation efficiency.
[Figure 9] Figure 9 shows the correlation between the lipid concentration and the light propagation efficiency.
[Figure 10] Figure 10 shows the correlation between the lipid concentration and the light propagation efficiency.
[Figure 11] Figure 11 schematically shows a particle concentration measurement device.
[Figure 12] Figure 12 shows the configuration of a control system of the particle concentration measurement device.
[Figure 13] Figure 13 shows correlation between the blood and a measured scattering coefficient in a case where the blood vessel depth and the lipid concentration change.
[Figure 14] Figure 14 is the flowchart of a particle concentration measurement method according to the embodiment.
[Figure 15] Figure 15 is the flowchart of a particle concentration measurement method according to an embodiment.

Description of Embodiment

An embodiment will be described below by using the drawings. The embodiment will be described with reference to an in-blood lipid concentration measurement device as a particle concentration measurement device. It is, however, noted that a measurement target is not limited to in-blood lipid, and it is assumed that the particle concentration of an arbitrary component in a living body can be measured.

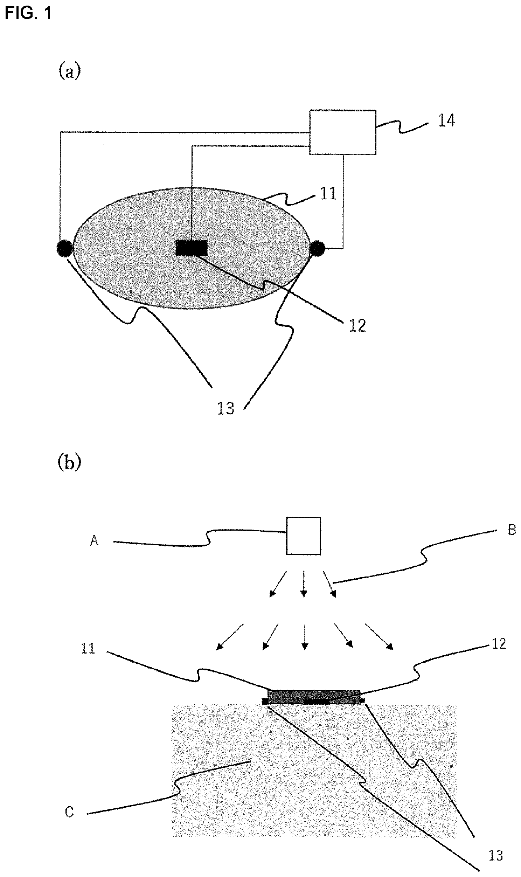

Figure 1 schematically shows an example of the configuration of a particle concentration measurement device 100 according to the embodiment. The particle concentration measurement device 100 includes a light blocking plate 11, a received light intensity detection unit 12, an incident light intensity detection unit 13, and a control unit 14, as shown in Figure 1.

The light blocking plate 11 in the embodiment is a black plastic plate and blocks periodic radiated light B, which is emitted from an illuminator A, with which a subject C is otherwise irradiated and which falls within a region from an irradiated-blocked boundary position to a light reception point. The shape and dimension of the light blocking plate 11 in the embodiment are those of an ellipse. The shape, dimension, and material of the light blocking plate 11 are not limited to those described above and only need to provide the function of blocking the radiated light B within a predetermined range around the received light intensity detection unit 12.

Blocking the periodic radiated light B from the illuminator A in the region from the irradiated-blocked boundary position to the light reception point allows selection and measurement of light having passed through the skin layer over a certain distance. The signal-to-noise ratio can thus be increased. Skillfully making the light from the illuminator A periodic or otherwise characteristic may allow measurement of even weak light in a proximate state. In this case, the light blocking plate 11 is not necessary.

The received light intensity detection unit 12 in the embodiment receives light emitted from the interior of the subject C to the exterior of the subject C. The received light intensity detection unit 12 in the embodiment is a photodiode. The received light intensity detection unit 12 is not limited to a photodiode and may instead be a CCD or a CMOS device. The received light intensity detection unit 12 may still instead be a component capable of receiving light having a wavelength so set as to belong to light other than the visible light. The received light intensity detection unit 12 is located roughly in a central portion of the light blocking plate 11. The received light intensity detection unit 12 is not necessarily located in the central portion, and a light blocking region only needs to be formed around the received light intensity detection unit 12. The received light intensity detection unit 12 is controlled by the control unit 14. The received light intensity detection unit 12 transmits sensed optical intensity to the control unit 14.

The incident light intensity detection unit 13 in the embodiment receives the light B incident from the exterior of the subject C into the subject C. The incident light intensity detection unit 13 in the embodiment is a photodiode. The incident light intensity detection unit 13 is not limited to a photodiode and may instead be a CCD or a CMOS device. The incident light intensity detection unit 13 may still instead be a component capable of receiving light having a wavelength so set as to belong to light other than the visible light. The incident light intensity detection unit 13 in the embodiment is so provided at the periphery of the light blocking plate 11 as to be separate by a predetermined distance from the received light intensity detection unit 12. The incident light intensity detection unit 13 is controlled by the control unit 14. The incident light intensity detection unit 13 transmits sensed optical intensity to the control unit 14.

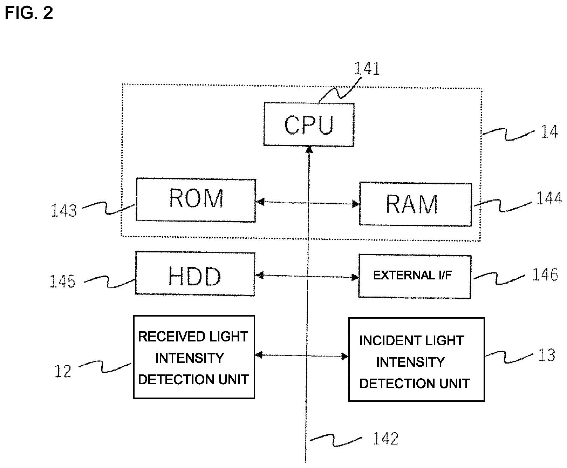

The configuration of a control system of the particle concentration measurement device 100 will next be described. Figure 2 is a block diagram of the particle concentration measurement device 100 according to the embodiment. A CPU (central processing unit) 141, a ROM (read only memory) 143, a RAM (random access memory) 144, an HDD (hard disk drive) 145, an external I/F (interface) 146, the received light intensity detection unit 12, and the incident light intensity detection unit 13 are connected to each other via a system bus 142. The CPU 141, the ROM 143, and the RAM 144 form the control unit 14.

The ROM 143 stores in advance a program executed by the CPU 141 and a threshold used by the CPU 141.

The RAM 144 has an area where the program executed by the CPU 141 is developed, a variety of memory areas, such as a work area where the program processes data, and other areas.

The HDD 145 stores data on a calibration curve created by correlating lipid concentration to light propagation efficiency for a plurality of persons.

Referring back to Figure 2, the external I/F 146 is an interface for communication with an external device, for example, a client terminal (PC). The external I/F 146 only needs to be an interface for data communication with an external device and may be an instrument (such as USB memory) to be locally connected to an external device or a network interface for communication via a network.

The particle concentration measurement device 100 having the configuration described above performs a light propagation measurement job based on a program set in advance.

In the embodiment, the light propagation is measured in a state in which the light source A, which emits periodic light, is not in contact with the subject C. A measurement error according to a measurement site is thus reduced.

In the approach of the related art, the light source is in close contact with a living body to cause the light to efficiently enter the living body. In a case where diffusion approximation, which is the measurement principle in the related art, is used, the light source is desirably in close contact with a subject also from the necessity for the light source to be a point light source or a light source considered to be a point light source.

The measurement principle of the related art based on diffusion approximation, however, causes a measurement value to vary in accordance with a measurement site because an actual living body is not a uniform system.

Further, the lipid measurement in the related art is verified by using near infrared light, which is called the window of a living body, in many cases. The reason for this is that near infrared light is likely to reach deep portions of a living body, and that the present invention attempts to use the characteristic.

In the approach of the related art, however, the fact that near infrared light reaches deep portions of a living body causes the near infrared light to be affected by complicated living body tissue, resulting, for example, in the measurement error according to a measurement site. It is believed that irradiating part of the living body with the light causes the measurement error.

In the embodiment, the effect of the complicated living body tissue is reduced by using, as the periodic light source A, light from an illuminator that is not provided in the particle concentration measurement device 100 but is separately provided in a room where the particle concentration measurement device 100 is installed, such as a radiator that radiates light over a wide range of a subject (for example, fluorescent lamp, LED, (diffused) laser, an incandescent lamp, an HID, and a halogen lamp. Even a nonperiodic light source, such as natural light (such as sunlight and moonlight) radiated over a wide range), may form periodic radiated light by providing the radiated light path with a mechanism that periodically opens and closes, such as a shutter, to adjust the light blocking period.

When an increase in the amount of in-blood lipid or the like increases the blood turbidity, the light is unlikely to enter the blood layer due to the difference in scattering coefficient between the blood and the peripheral tissue (assumed to be a skin layer).

Even in measurement in a uniform system, a decrease in the turbidity tends to increase forward scattered light, whereas an increase in the turbidity tends to relatively increase back scattered light.

In the case of a two-layer system formed of the skin layer and the blood layer, and when the blood turbidity increases in a layer that is separate from the light source and deep in the living body (assumed to be a blood layer), lateral scattering increases. When observed from the side facing the light incident surface, the light reachable range widens, and the incident light therefore efficiently propagates far.

The skin layer used herein is a layer which spreads over a wide range and where the blood is hardly exists, in other words, a layer that does not bleed even when injured. A blood vessel layer is a site where the blood concentrates, such as a vein, or a widely spread layer that is present around a vein and includes capillary blood vessels and the like. A difference in refractive index between the two layers described above occurs due to the difference in blood density and causes reflection at the interface between the two layers. In other words, as the capillary blood vessels and veins mix each other in a deep portion and the density difference decreases accordingly, the deep portion can be considered as a uniform layer. The skin layer can also be interpreted as the outermost layer of the skin, and the blood layer can be considered as the dermis and layers deeper than the dermis. The definition of the skin layer and the blood layer in the present invention is based on the optical characteristics of a layer that are measurable with a measurement instrument and based further on evaluation of whether or not blood turbidity can be measured.

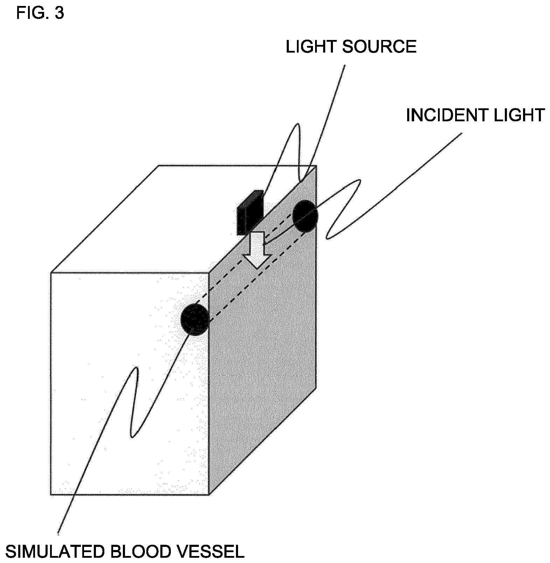

An experiment shown in Figure 3 was conducted to verify the definition. Different types of blood in terms of turbidity were injected into a simulated blood vessel. As for the blood turbidity, it is known that the in-blood neutral fat concentration varies and the variance in neutral fat concentration causes the turbidity to vary.

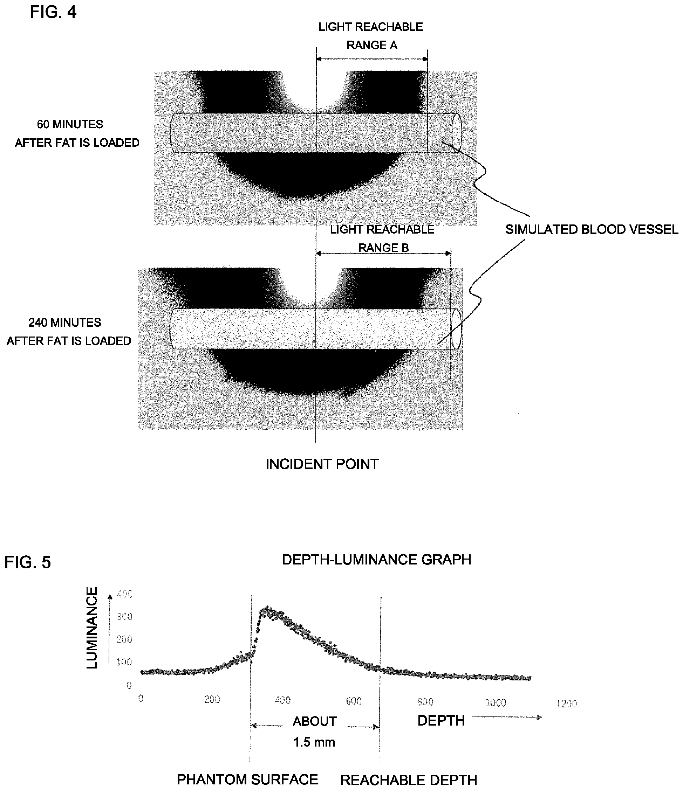

In a case where a layer in which the turbidity varies due to the blood (blood layer), such as a blood vessel, and the skin layer that is not affected by the blood fall within the light reachable depth range, the experiment proved that an increase in the turbidity in the blood layer causes the skin layer to form a light propagating optical path and the light therefore propagates far (Figure 4). It is believed that the phenomenon described above is based on the fact that the increase in the turbidity in the deep portion increases turbulence of diffusive reflection in the surface (interface) reflection and the angle of reflection increases accordingly.

As described above, to measure the light propagation efficiency determined by a change in the blood turbidity in a living body, it is desirable that the light radiation depth falls within the skin layer and the thickness of the skin layer is smaller than or equal to 3 mm.

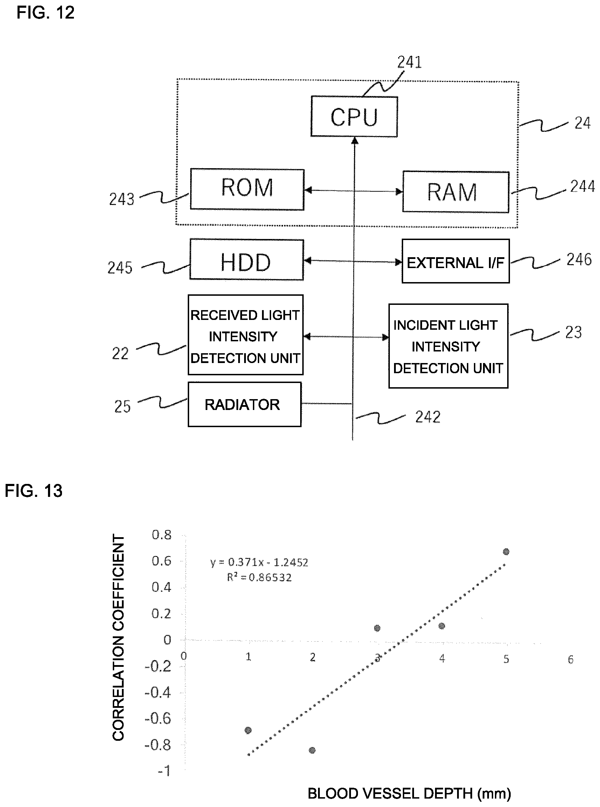

Figure 13 shows correlation between the blood and the measured scattering coefficient in a case using a simulated living body where the blood vessel depth and the lipid concentration change, and negative correlation (high light propagation efficiency) is achieved when the blood vessel is located in a shallow position. It is believed that the diffusion theory for a uniform system is applied so that positive correlation is achieved at a depth of 5 mm where the peripheral tissue and blood vessels are averaged, and that the positive and negative phenomena mix with each other at depths ranging from 3 to 4 mm.

When a vein or the like is present at a location where the outermost layer of the skin is thin, the light having entered the blood vessel is scattered in the blood vessel, resulting in an increase in the optical path of the light. The absorption effect of the blood is therefore enhanced, so that the light reachable range narrows, resulting in the correlation opposite the correlation in the skin layer measurement. In this case, providing information on blood absorption and the like allows in-advance expectation of negative correlation. The situation can therefore be handled by in-advance correction.

To provide information on a change in the light propagation efficiency in the skin layer, light from a fluorescent lamp was used as the illumination light, and the device shown in Figure 3 was used to measure the received light intensity.

The intensity of the radiated light in a case where a fluorescent lamp having a power of 32 W was separate from the measurement point by 2 m was 0.0025 ($mW/mm^2$), and the reachable depth in phantom measurement was 1.5 mm (Figure 5). In actual measurement, an effective reachable depth is believed to be 0.75 mm, which is half the reachable depth described above, in consideration of the fact that the light makes a round trip along the optical path.

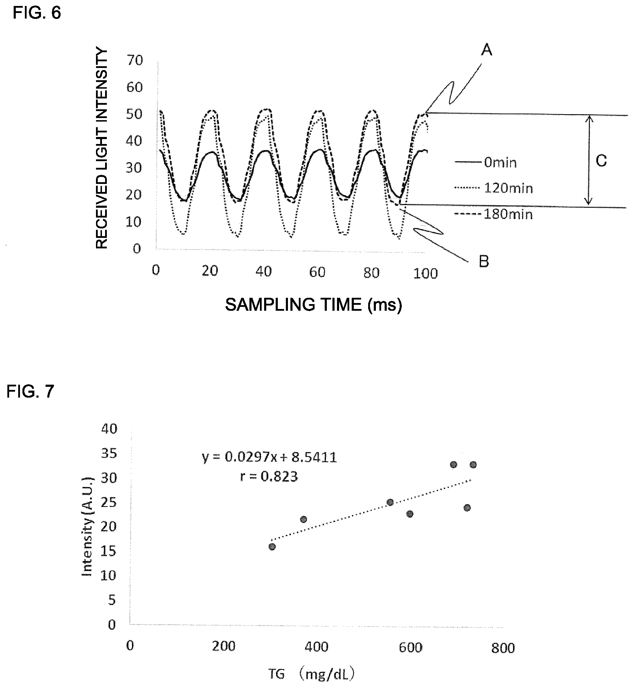

Figure 6 shows the result of the measurement of the received light intensity in the skin layer. The cycle of the observed received light intensity is the cycle of 50 Hz at which the fluorescent lamp emits light. The amplitude (or total amplitude (also called both amplitude)) of the received light intensity at the time of hunger (0 min in Figure 6) changes as the measurement period elapses and increases as TG rises (120 min, 180 min in Figure 6), as shown in Figure 6. This is a phenomenon showing that the turbidity in the blood layer, such as a blood vessel, increases so that it is difficult for the light to propagate in the blood layer, resulting in an increase in the light propagation efficiency in the skin layer where the turbidity is relatively low. The term "total amplitude" is in general a peak-to-peak value of a wave and is typically twice the amplitude of the wave. The term "total amplitude" is also called an "both amplitude."

Assuming that the measurement target is two layers, the skin layer and the blood layer, and that the amount of change in the scattering coefficient in the skin layer, which is shallower than the blood layer, is negligible (smaller change than in the blood layer because the blood density is lower), it is believed that the light reflected off the blood layer propagates in the skin layer, where the scattering coefficient is relatively low.

Further, since illumination light is used, a measured value is small, but the light does not reach deep portions of the living body, and the light propagates via the entire periphery of the device in Figure 1, it is believed that information on the tissue in the vicinity of the skin is averaged and transmitted to the light receiver.

Further, it is believed based on the frequency that the light from the fluorescent lamp is received.

That is, the in-blood lipid concentration increases, resulting in an increase in the propagation efficiency in the skin layer.

The lipid concentration used herein refers to a change in the average particle diameter of lipid particles, such as in-blood chylomicron and VLDL, or a change in the number of lipid particles and further refers to a change associated with a phenomenon in which a change in the lipid particle concentration increases the blood scattering coefficient and affects the received light intensity.

The control unit 14 calculates the total amplitude from the received light intensity sensed by the received light intensity detection unit 12. The total amplitude is calculated from the received light intensity in the embodiment, and the amplitude, which is half the total amplitude, may instead be calculated from the received light intensity.

The total amplitude can be calculated by the following expression:

```
Total amplitude = received light intensity tp (mV) -

received light intensity tb (mV)
```

The received light intensity tp (mV) represents the received light intensity at a peak top of a received light intensity change that occurs when the illumination light periodically changes. The character A in Figure 6 corresponds to the received light intensity tp in the received light change for 180 min. The received light intensity tb (mV) represents the received light intensity at the bottom of the periodic change in the received light intensity. The character B in Figure 6 corresponds to the received light intensity tb in the received light change for 180 min. The character C in Figure 6 therefore represents the total amplitude in the received light change for 180 min. The character tp represents the point of time when the change in the received light intensity reaches the peak top. The character tb represents the point of time when the change in the received light intensity reaches the bottom. The time interval between tp and tb is half the cycle of the periodic change in the illumination light.

For example, when room light is used and a plurality of light sources are present, a waveform with a shoulder is formed, for example, due to the difference in intensity of the light emitted from the light sources. Also in this case, the peak top and the peak bottom in the measurement may be employed, as described above.

To calculate the amount of periodic change, the difference between the average and the peak top or the difference between the average and the peak bottom, that is, a half maximum may be used to perform the analysis.

The control unit 14 calculates the light propagation efficiency from the total amplitude. The light propagation efficiency refers to a value representing how intense light having a certain intensity at the time of radiation is at a certain light reception point. Even in a case where the specific value of the intensity at the time of radiation is unknown, it can be considered that the light propagation efficiency has changed when the intensity at the time of radiation is fixed and the received light intensity changes. The light propagation efficiency is calculated from the total amplitude in the embodiment, and the light propagation efficiency may instead be calculated from the amplitude, which is half the total amplitude.

The light propagation efficiency can be calculated by the following expression.

(1) A case where the intensity of the incident light is known (that is, a case where the incident light intensity detection unit 13 is provided)

```
Light propagation efficiency = total amplitude A / total amplitude B * 100
```

The total amplitude A is the total amplitude of the received light intensity and can be calculated by the following expression:

$$\text{Total amplitude A} = \text{received light intensity tp (mV)}$$
$$- \text{received light intensity tb (mV) (intensity of light}$$
$$\text{received by received light intensity detection unit 12)}$$

The total amplitude B is the total amplitude of the incident light intensity and can be calculated by the following expression:

$$\text{Total amplitude B} = \text{incident light intensity tp (mV)}$$
$$- \text{incident light intensity tb (mV) (intensity of light}$$
$$\text{received by incident light intensity detection unit 13)}$$

The character tp represents the point of time when the received light intensity or the incident light intensity reaches the peak top. The character tb represents the point of time when the received light intensity or the incident light intensity reaches the bottom. The time interval between tp and tb is half the cycle of the periodic change in the illumination light.

(2) A case where the intensity of the incident light is unknown (that is, a case where the incident light intensity detection unit 13 is not provided)

$$\text{Light propagation efficiency} = \text{total amplitude T1} -$$
$$\text{total amplitude T2}$$

The total amplitude T1 is the total amplitude of the received light intensity at measurement time T1 (first point of time) and can be calculated by the following expression:

$$\text{Total amplitude T1} = \text{received light intensity T1}$$
$$\text{(tp) (mV)} - \text{received light intensity T1 (tb) (mV)}$$
$$\text{(intensity of light received by received light intensity}$$
$$\text{detection unit 12)}$$

The character tp represents the point of time when the received light intensity or the incident light intensity reaches the peak top at the measurement time T1. The character tb represents the point of time when the received light intensity or the incident light intensity reaches the bottom at the measurement time T1.
The total amplitude T2 is the total amplitude of the received light intensity at measurement time T2 (second point of time) and can be calculated by the following expression:

$$\text{Total amplitude T2} = \text{received light intensity T2}$$

$$\text{(tp) (mV)} - \text{received light intensity T2 (tb) (mV)}$$

$$\text{(intensity of light received by received light intensity}$$

$$\text{detection unit 12)}$$

The character tp represents the point of time when the received light intensity or the incident light intensity reaches the peak top at the measurement time T2. The character tb represents the point of time when the received light intensity or the incident light intensity reaches the bottom at the measurement time T2.

T1 and T2 used herein are each a point of time when the measurement is made. T1 and T2 are each a point of time when the measurement is made and which falls within a range of several minutes to several hours, such as before or after meals. For example, with the point of time of hunger (T1) defined as a start point, how much the total amplitude has been changed after a predetermined period elapses (T2) is calculated. For example, assuming that the total amplitude at 0 minutes (T1) is 25 a.u. and the total amplitude at 180 minutes (T2) is 45 a.u., the light propagation efficiency is 20 a.u., which is the difference between the amplitudes described above.

The intensity at the time of radiation and the incident light intensity are the intensity at the skin surface and the intensity after the light enters the skin, respectively, in the exact sense, but can be practically considered to be the same value (handled as measured values).

The received light intensity used to calculate the light propagation efficiency may, for example, be the height of the amplitude or the total amplitude when a light source that emits periodic light is used. In this case, the light propagation efficiency can be determined from the received light intensity, and a secondary effect of avoiding the effects of other types of continuous light can also be provided.

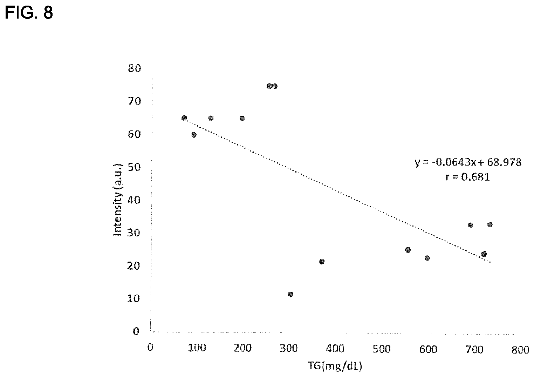

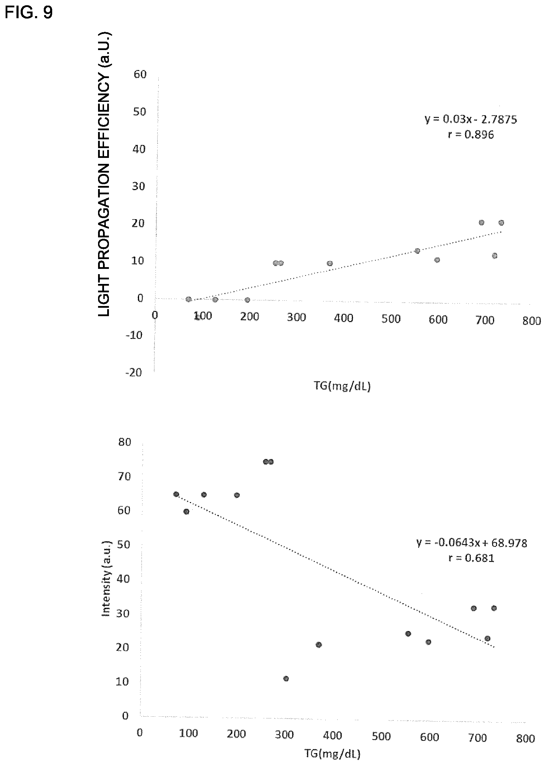

The control unit 14 calculates the lipid concentration from the light propagation efficiency. The calculation method includes creating correlation between the lipid concentration and the light propagation efficiency for a plurality of persons, such as those shown in Figures 7 to 10, and holding data on the correlation in the form of a calibration curve in the HDD 145, and the control unit 14 calculates the light propagation efficiency or the lipid concentration corresponding to the light propagation efficiency from the data.

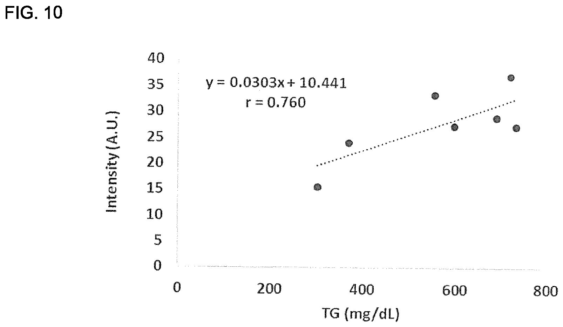

Figures 7 to 10 show the correlation between the TG concentration and the light propagation efficiency. Figures 7 to 9 show the correlation in a portion in the vicinity of a vein at the elbow bone in the forearm, and Figure 10 shows the correlation in a portion slightly below the wrist. The correlation coefficient is greater than or equal to 0.75 in each of the two sites, meaning that strong correlation can be ascertained.

A subject in the present test can visually see no vein clearly, and it is therefore believed that the depth of the vein is at least 1 mm or greater from the skin surface.

In an example using a living body, a relative change in the light propagation efficiency in the skin layer is measured by adjusting the light reachable depth to a small value, whereas in a practical example, the result of the measurement of the simulated living body shown in Figure 4 shows that the light propagation efficiency in the skin layer can be measured even when the intensity at the time of radiation is increased.

For example, the light propagation efficiency can be measured even in a portion where blood vessels gather, such as a fingertip.

In the embodiment, the received light intensity detection unit and the control unit are integrated each other into a single unit, but not necessarily. For example, a sensor (such as CMOS device) provided in a user device, such as a mobile terminal (smartphone, tablet terminal, and mobile PC) may be used as the received light intensity detection unit, and a control unit may be provided, for example, in a server device connected to the user device over a network.

The particle concentration measurement device according to the embodiment is used under periodic illumination light, for example, in a room and is communicably connected to a user device including a received light intensity detection unit that detects the intensity of the light emitted from a subject and received by the received light intensity detection unit. The particle concentration measurement device includes a control unit that determines the total amplitude from the received light intensity transmitted from the user device, determines the light propagation efficiency from the total amplitude, and calculates the concentration of the particles in the subject from the light propagation efficiency.

The user device may further include an incident light intensity detection unit that detects the intensity of incident light incident on a subject receiving periodic illumination light, for example, in a room, and the control unit may determine the total amplitude from the incident light intensity, determine the light propagation efficiency from the total amplitude of the received light intensity and the total amplitude of the incident light intensity, and calculate the concentration of the particles in the subject from the light propagation efficiency.

The received light intensity detection unit may detect the intensity of the light radiated from the subject and received by the received light intensity detection unit at a first point of time and a second point of time separate from each other by a predetermined time interval, and the control unit may determine the light propagation efficiency from the total amplitude at the first point of time and the total amplitude at the second point of time and calculate the concentration of the particles in the subject from the light propagation efficiency.

A light blocker that blocks the light toward the surface of the subject may further be provided around the received light intensity detection unit. The particle concentration may be the lipid concentration. The specific contents of the processes described above are the same as the contents of the processes carried out by the particle concentration measurement device described above and are therefore not described.

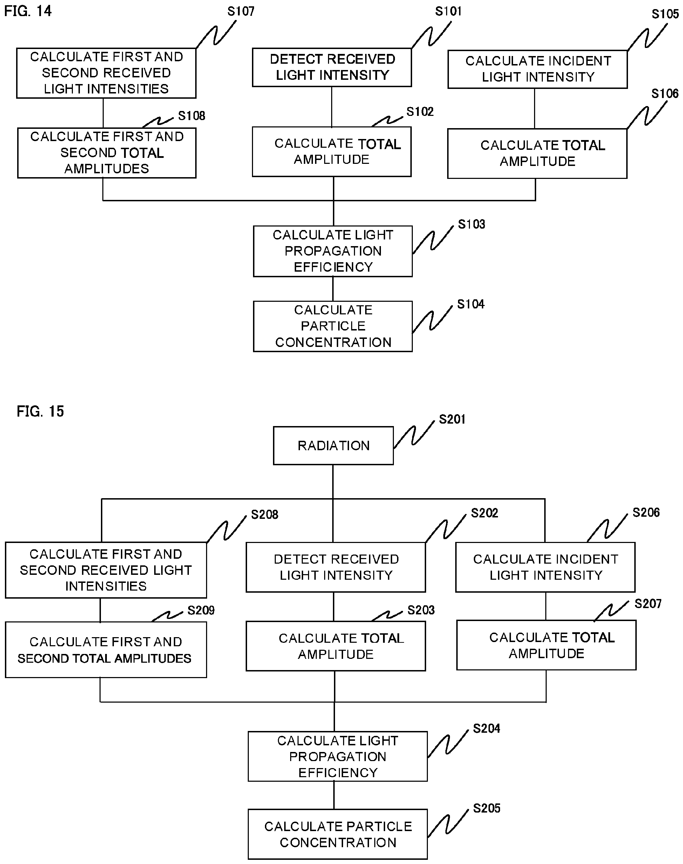

A particle concentration measurement method according to the embodiment will next be described. Figure 14 is the flowchart of the particle concentration measurement method according to the embodiment.

The particle concentration measurement method according to the embodiment is a particle concentration measurement method performed under periodic illumination light. The received light intensity detection unit detects the intensity of the light emitted from the subject and received by the received light intensity detection unit (step S101), and the control unit determines the total amplitude from the received light intensity (step S102), determines the light propagation efficiency from the total amplitude (step S103), and calculate the concentration of the particles in the subject from the light propagation efficiency (step S104) .

When the particle concentration measurement device includes the incident light intensity detection unit, the incident light intensity detection unit may detect the intensity of incident light incident on a subject that receives the periodic illumination light (step S105), and the control unit may determine the total amplitude from the incident light intensity (step S106), determine the light propagation efficiency from the total amplitude of the received light intensity and the total amplitude of the incident light intensity (step S103), and calculate the concentration of the particles in the subject from the light propagation efficiency (step S104).

The received light intensity detection unit may detect the intensity of the light emitted from the subject and received by the received light intensity detection unit at a first point of time and a second point of time separate from each other by a predetermined time interval (step S107), and the control unit may calculate the total amplitude at the first point of time and the total amplitude at the second point of time from the received light intensities at the first and second points of time (step S108), determine the light propagation efficiency from the total amplitude at the first point of time and the total amplitude at the second point of time (step S103), and calculate the concentration of the particles in the subject from the light propagation efficiency (step S104).

A light blocker that blocks the light toward the surface of the subject may further be provided around the received light intensity detection unit, and the particle concentration may be the lipid concentration. The specific contents of the processes described above are the same as the contents of the processes carried out by the particle concentration measurement device described above and are therefore not described.

A particle concentration measurement program according to the embodiment will next be described.

A device is used under periodic illumination light and is communicably connected to a user device including a received light intensity detection unit that detects the intensity of the light emitted from a subject and received by the received light intensity detection unit.

The particle concentration measurement program according to the embodiment causes a computer of the device to carry out the process of determining the total amplitude from the received light intensity transmitted from the user device and calculating the concentration of the particles in the subject from the total amplitude.

The particle concentration measurement program according to the embodiment causes the computer of the device to carry out the process of determining the total amplitude from the received light intensity transmitted from the user device, determining the light propagation efficiency from the total amplitude, and calculating the concentration of the particles in the subject from the light propagation efficiency.

When the user device further includes an incident light intensity detection unit that detects the intensity of incident light incident on a subject as part of illumination light or natural light, the particle concentration measurement program according to the embodiment may cause the computer of the device to carry out the process of determining the total amplitude from the incident light intensity, determining the light propagation efficiency from the total amplitude of the received light intensity and the total amplitude of the incident light intensity, and calculating the concentration of the particles in the subject from the light propagation efficiency.

The particle concentration measurement program according to the embodiment may cause the received light intensity detection unit to detect the intensity of the light emitted from the subject and received by the received light intensity detection unit at a first point of time and a second point of time separate from each other by a predetermined time interval and cause the computer of the device to carry out the process of determining the total amplitude at the first point of time and the total amplitude at the second point of time from the received light intensities at the first and second points of time, determining the light propagation efficiency from the total amplitude at the first point of time and the total amplitude at the second point of time, and calculate the concentration of the particles in the subject from the light propagation efficiency.

A light blocker that blocks the light toward the surface of the subject may further be provided around the received light intensity detection unit, and the particle concentration may be the lipid concentration. The specific contents of the processes are the same as the contents of the processes in the embodiment described above and are therefore not described.

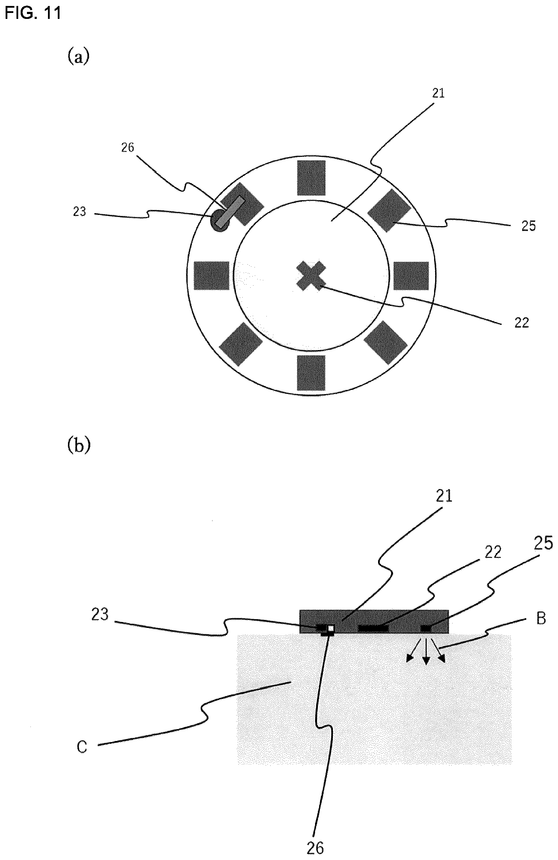

Figure 11 schematically shows an example of the configuration of a particle concentration measurement device 200 according to another embodiment. The configuration and processes common to those of the particle concentration measurement device 100 according to the embodiment described above are not described. The particle concentration measurement device 200 includes a light blocking plate 21, a received light intensity detection unit 22, an incident light intensity detection unit 23, a control unit 24, radiators 25, and a mirror 26, as shown in Figure 11.

The light blocking plate 21 in the embodiment is a black plastic plate and blocks radiated light with which a subject is otherwise irradiated and which falls within a region from an irradiated-blocked boundary position to a light reception point. The shape and dimension of the light blocking plate 21 in the embodiment are those of an ellipse. The shape, dimension, and material of the light blocking plate 21 are not limited to those described above and only need to provide the function of blocking the radiated light within a predetermined range of the subject.

Blocking the radiated light within the region from the irradiated-blocked boundary position to the light reception point allows selection and measurement of light having passed through the skin layer over a certain distance. The signal-to-noise ratio can thus be increased. Skillfully making the light periodic or otherwise characteristic may allow measurement of even weak light in a proximate state. In this case, the light blocking plate 21 is not necessary.

The received light intensity detection unit 22 in the embodiment receives light emitted from the interior of the subject to the exterior of the subject. The received light intensity detection unit 22 in the embodiment is a photodiode. The received light intensity detection unit 22 is not limited to a photodiode and may instead be a CCD or a CMOS device. The received light intensity detection unit 22 may be a component capable of receiving light having a wavelength so set as to belong to light other than the visible light. The received light intensity detection unit 22 is located in a central portion of the light blocking plate 21. The received light intensity detection unit 22 is not necessarily located in the central portion, and a light blocking region only needs to be formed around the received light intensity detection unit 22. The received light intensity detection unit 22 is controlled by the control unit 24. The received light intensity detection unit 22 transmits sensed optical intensity to the control unit 24.

The incident light intensity detection unit 23 in the embodiment receives the light radiated from the radiators 25. The incident light intensity detection unit 23 is a photodiode. The incident light intensity detection unit 23 is not limited to a photodiode and may instead be a CCD or a CMOS device. The wavelength may be so set as to belong to light other than the visible light, and light having the thus set wavelength may be received. At least one of the plurality of radiators 25 is provided with the incident light intensity detection unit 23. The incident light intensity detection unit 23 is controlled by the control unit 24. The incident light intensity detection unit 23 transmits sensed optical intensity to the control unit 24.

The radiators 25 in the embodiment are arranged at a plurality of locations along a circumference roughly around the received light intensity detection unit 22 and are separate by a predetermined distance from the received light intensity detection unit 12. The arrangement of the radiators 25 is not limited to the arrangement described above and may be any arrangement that allows light radiation over a wide range. The radiators 25 are each, for example, a fluorescent lamp, an LED, a laser, a white incandescent lamp, an HID, or a halogen lamp. The radiators 25 may each be provided with a mechanism that periodically opens and closes, such as a shutter, to adjust the light blocking period so that the radiated light forms periodic light. The illuminance of the light radiated from each of the radiators 25 is controlled by the control unit 25. The intensity at the time of radiation may be increased to allow the device to target a vein or search for information on the position of the vein.

The light irradiated area may be roughly equal to the surface area of the LED but is preferably larger.

As described above, to measure the light propagation efficiency determined by a change in the blood turbidity in a living body, it is desirable to cause the depth reached by the radiated light to fall within the skin layer. It is therefore preferable that the depth reached by the light radiated from the radiators 25 is set at about 1 mm.

Therefore, when the skin layer is the measurement target, the radiators 25 that emit light having an adjusted intensity at the time of radiation may be in close contact with the skin layer.

The mirror 26 in the present embodiment is provided between the incident light intensity detection unit 23 and one of the radiators 25. The mirror 26 is a mechanism for measuring the incident light intensity and ensuring the accuracy thereof because the incident light slightly changes due, for example, to a change in voltage.

The configuration of a control system of the particle concentration measurement device 200 will next be described. Figure 12 is a block diagram of the particle concentration measurement device 200 according to the embodiment. A CPU (central processing unit) 241, a ROM (read only memory) 243, a RAM (random access memory) 244, an HDD (hard disk drive) 245, an external I/F (interface) 246, the received light intensity detection unit 22, the incident light intensity detection unit 23, and the radiators 25 are connected to each other via a system bus 242. The CPU 241, the ROM 243, and the RAM 244 form the control unit 24.

The ROM 243 stores in advance a program executed by the CPU 241 and a threshold used by the CPU 241.

The RAM 244 has an area where the program executed by the CPU 241 is developed, a variety of memory areas, such as a work area where the program processes data, and other areas.

The HDD 245 stores data on a calibration curve created by correlating lipid concentration to the light propagation efficiency or the amount of change in the light propagation efficiency for a plurality of persons.

The external I/F 246 is an interface for communication with an external device, for example, a client terminal (PC). The external I/F 246 only needs to be an interface for data communication with an external device and may be an instrument (such as USB memory) to be locally connected to an external device or a network interface for communication via a network.

The particle concentration measurement device 200 having the configuration described above performs a light propagation measurement job based on a program set in advance.

The radiators 25 radiate light circumferentially toward the surface of the subject. In the embodiment, illumination light or natural light is used, whereas in other embodiments, illumination light that reaches a subject to a depth of about 1 mm is radiated via the circumference of the subject.

The control unit 24 calculates the light propagation efficiency from the optical intensity sensed by the received light intensity detection unit 22 and the optical intensity sensed by the incident light intensity detection unit 23. The method for calculating the light propagation efficiency is the same as the method in the embodiment described above.

The control unit 24 calculates the lipid concentration from the light propagation efficiency. The method for calculating the lipid concentration is the same as the method in the embodiment described above.

In the embodiment, the radiators, the received light intensity detection unit, and the control unit are integrated with one another into a single unit, but not necessarily. For example, an illuminator (such as LED) and a sensor (such as CMOS device) provided in a user device, such as a mobile terminal (smartphone, tablet terminal, and PC) as the radiators and the received light intensity detection unit may be used, and a control unit may be provided in a server device connected to the user device over a network.

The particle concentration measurement device according to the embodiment is communicably connected to the user device including a radiator that radiates light to the subject and a received light intensity detection unit that is so disposed as to be separate from the radiator by a predetermined distance and detects the intensity of the light emitted from the subject and received by the received light intensity detection unit. The particle concentration measurement device includes a control unit that determines the light propagation efficiency from the received light intensity transmitted from the user device and calculates the concentration of the particles in the subject from the light propagation efficiency.

The user device may further include an incident light intensity detection unit that detects the intensity of incident light incident on a subject from the radiator, and the control unit may determine the total amplitude from the incident light intensity, determine the light propagation efficiency from the total amplitude of the received light intensity and the total amplitude of the incident light intensity, and calculate the concentration of the particles in the subject from the light propagation efficiency.

The received light intensity detection unit may detect the intensity of the light emitted from the subject and received by the received light intensity detection unit at a first point of time and a second point of time separate from each other by a predetermined time interval, and the control unit may determine the light propagation efficiency from the difference in the total amplitude between the first point of time and the second point of time and calculate the concentration of the particles in the subject described above from the light propagation efficiency.

A light blocker that blocks the light toward the surface of the subject may further be provided around the received light intensity detection unit. The particle concentration may be the lipid concentration. The specific contents of the processes described above are the same as the contents of the processes carried out by the particle concentration measurement device according to the embodiment described above and are therefore not described.

A particle concentration measurement method according to another embodiment will next be described. Figure 15 is the flowchart of the particle concentration measurement method according to the other embodiment.

The particle concentration measurement method according to the other embodiment includes causing the radiator to radiate periodic light to a the subject in a predetermined radiation position (step S201), causing the received light intensity detection unit to detect the intensity of the light emitted from the subject and received by the received light intensity detection unit in a position separate from the radiation position by a predetermined distance (step S202), causing the control unit to determine the light propagation efficiency from the received light intensity (step S203), causing the control unit to determine the light propagation efficiency from the total amplitude (step S204), and causing the control unit to calculate the concentration of the particles in the subject from the light propagation efficiency (step S205) .

When the particle concentration measurement device includes the incident light intensity detection unit, the incident light intensity detection unit may detect the intensity of incident light incident on the subject from the radiation position (step S206), and the control unit may determine the total amplitude from the incident light intensity (step S207), determine the light propagation efficiency from the total amplitude of the received light intensity and the total amplitude of the incident light intensity (step S204), and calculate the concentration of the particles in the subject from the light propagation efficiency (step S205).

The received light intensity detection unit may detect the intensity of the light emitted from the subject and received by the received light intensity detection unit at a first point of time and a second point of time separate from each other by a predetermined time interval (step S208), and the control unit may calculate the total amplitude received light intensity at the first point of time and the total amplitude at the second point of time from the received light intensities at the first and second points of time (step S209), determine the light propagation efficiency from the total amplitude at the first point of time and the total amplitude at the second point of time (step S204), and calculate the concentration of the particles in the subject from the light propagation efficiency (step S205).

A light blocker that blocks the light toward the surface of the subject may further be provided around the received light intensity detection unit, and the particle concentration may be the lipid concentration. The specific contents of the processes described above are the same as the contents of the processes carried out by the particle concentration measurement device according to the embodiment described above and are therefore not described.

A particle concentration measurement program according to another embodiment will next be described.

A device is communicably connected to a user device including a radiator that radiates light to a subject and a received light intensity detection unit that is disposed in a position separate from the radiator by a predetermined distance and detects the intensity of the light emitted from the subject and received by the received light intensity detection unit.

The particle concentration measurement program according to the embodiment causes a computer of the device to carry out the process of determining the total amplitude from the received light intensity transmitted from the user device and calculating the concentration of the particles in the subject from the total amplitude.

The particle concentration measurement program according to the embodiment causes the computer of the device to carry out the process of determining the total amplitude from the received light intensity transmitted from the user device, determining the light propagation efficiency from the total amplitude, and calculating the concentration of the particles in the subject from the light propagation efficiency.

When the user device further includes an incident light intensity detection unit that detects the intensity of incident light incident on the subject from the radiator, the particle concentration measurement program according to the embodiment may cause the computer of the device to carry out the process of determining the total amplitude from the incident light intensity, determining the light propagation efficiency from the total amplitude of the received light intensity and the total amplitude of the incident light intensity, and calculating the concentration of the particles in the subject from the light propagation efficiency.

The particle concentration measurement program according to the embodiment may cause the received light intensity detection unit to detect the intensity of the light emitted from the subject and received by the received light intensity detection unit at a first point of time and a second point of time separate from each other by a predetermined time interval and cause the computer of the device to carry out the process of calculating the total amplitude at the first point of time and the total amplitude at the second point of time from the received light intensities at the first and second points of time, determining the amount of change in the light propagation efficiency from the total amplitude at the first point of time and the total amplitude at the second point of time, and calculating the concentration of the particles in the subject from the amount of change in the light propagation efficiency.

A light blocker that blocks the light toward the surface of the subject may further be provided around the received light intensity detection unit, and the particle concentration may be the lipid concentration. The specific contents of the processes described above are the same as the contents of the processes in the embodiment described above and are therefore not described.

The embodiments have been described above but have been presented by way of example and are not intended to limit the scope of the present invention. The novel embodiments can be implemented in a variety of other forms, and a variety of omissions, replacements, and changes can be made to the embodiments to the extent that they do not depart from the substance of the present invention. The embodiments and variations fall within the scope and substance of the present invention and also fall within the inventions set forth in the claims and the scope equivalent thereto.

Reference Sings list

100    Particle concentration measurement device
12     Received light intensity detection unit
13     Incident light intensity detection unit
14     Control unit

**Claims**

1. A particle concentration measurement device used under temporally periodic illumination light, the particle concentration measurement device comprising:

   a received light intensity detection unit that detects a received light intensity of light emitted from a subject; and
   a control unit that determines an amplitude from the received light intensity and calculates a concentration of particles in the subject from the amplitude.

2. The light propagation measurement devices according to claim 1, wherein the control unit determines light propagation efficiency from the amplitude and calculates the concentration of the particles in the subject from the light propagation efficiency.

3. The particle concentration measurement device according to claim 2,
   further comprising an incident light intensity detection unit that detects an incident light intensity of light incident on the subject from the illumination light,
   wherein the control unit determines the amplitude from the incident light intensity and determines the light propagation efficiency from the amplitude of the received light intensity and the amplitude of the incident light intensity.

4. The particle concentration measurement device according to claim 2,
   wherein the received light intensity detection unit detects the received light intensity of the light emitted from the subject at a first point of time and a second point of time separate from each other by a predetermined time interval, and
   the control unit determines the light propagation efficiency from the amplitude of the received light intensity at the first point of time and the amplitude of the received light intensity at the second point of time.

5. A particle concentration measurement device comprising:

   a radiator that radiates temporally periodic light to a subject;
   a received light intensity detection unit that is so disposed as to be separate from the radiator by a predetermined distance and detects a received light intensity of light emitted from the subject; and
   a control unit that determines an amplitude from the received light intensity and calculates a concentration of particles in the subject from the amplitude.

6. The particle concentration measurement device according to claim 5, wherein the control unit determines light propagation efficiency from the amplitude and calculates the concentration of the particles in the subject from the light propagation efficiency.

7. The particle concentration measurement device according to claim 6,
   further comprising an incident light intensity detection unit that detects an incident light intensity of light incident on the subject from the radiator,
   wherein the control unit determines the amplitude from the incident light intensity and determines the light propagation efficiency from the amplitude of the received light intensity and the amplitude of the incident light intensity.

8. The particle concentration measurement device according to claim 6,
   wherein the received light intensity detection unit detects the received light intensity of the light emitted from the subject at a first point of time and a second point of time separate from each other by a predetermined time interval, and
   wherein the control unit determines the light propagation efficiency from the amplitude of the received light intensity at the first point of time and the amplitude of the received light intensity at the second point of time.

9. A particle concentration measurement device communicably connected to a user device used under temporally periodic illumination light and including a received light intensity detection unit that detects a received light intensity of light emitted from a subject, the particle concentration measurement device comprising
   a control unit that determines an amplitude from the received light intensity transmitted from the user device and calculates a concentration of particles in the subject from the amplitude.

10. The particle concentration measurement device according to claim 9, wherein the control unit determines light propagation efficiency from the amplitude and calculates the concentration of the particles in the subject from the light propagation efficiency.

**11.** The particle concentration measurement device according to claim 10,
wherein the user device further includes an incident light intensity detection unit that detects an incident light intensity of light incident on the subject from the illumination light, and
the control unit determines the amplitude from the incident light intensity and determines the light propagation efficiency from the amplitude of the received light intensity and the amplitude of the incident light intensity.

**12.** The particle concentration measurement device according to claim 10,
wherein the received light intensity detection unit detects the received light intensity of the light emitted from the subject at a first point of time and a second point of time separate from each other by a predetermined time interval, and
the control unit determines the light propagation efficiency from the amplitude of the received light intensity at the first point of time and the amplitude of the received light intensity at the second point of time.

**13.** A particle concentration measurement device communicably connected to a user device including a radiator that radiates temporally periodic light to a subject and a received light intensity detection unit that is so disposed as to be separate from the radiator by a predetermined distance and detects a received light intensity of light emitted from the subject, the particle concentration measurement device comprising
a control unit that determines an amplitude from the received light intensity transmitted from the user device and calculates a concentration of particles in the subject from the amplitude.

**14.** The particle concentration measurement device according to claim 13, wherein the control unit determines light propagation efficiency from the amplitude and calculates the concentration of the particles in the subject from the light propagation efficiency.

**15.** The particle concentration measurement device according to claim 14,
wherein the user device further includes an incident light intensity detection unit that detects an incident light intensity of light incident on the subject from the radiator, and
the control unit determines the amplitude from the incident light intensity and determines the light propagation efficiency from the amplitude of the received light intensity and the amplitude of the incident light intensity.

**16.** The particle concentration measurement device according to claim 14,
wherein the received light intensity detection unit detects the received light intensity of the light emitted from the subject at a first point of time and a second point of time separate from each other by a predetermined time interval, and
the control unit determines the light propagation efficiency from the amplitude of the received light intensity at the first point of time and the amplitude of the received light intensity at the second point of time.

**17.** The particle concentration measurement device according to any of claims 1 to 16, further comprising a light blocker that is disposed around the received light intensity detection unit and blocks light to a surface of the subject.

**18.** The particle concentration measurement device according to any of claims 1 to 17, wherein the particle concentration is lipid concentration.

**19.** A particle concentration measurement method performed under temporally periodic illumination light, the method comprising:

detecting a received light intensity of light emitted from a subject; and
determining an amplitude from the received light intensity and calculating a concentration of particles in the subject from the amplitude.

**20.** A particle concentration measurement method comprising:

radiating temporally periodic light to a subject;
detecting a received light intensity of light emitted from the subject in a position separate from the radiation position by a predetermined distance; and
determining an amplitude from the received light intensity and calculating a concentration of particles in the subject from the amplitude.

**21.** A particle concentration measurement program that causes a computer of a device communicably connected to a user device used under temporally periodic illumination light and including a received light intensity detection unit that detects a received light intensity of light emitted from a subject to carry out the process of determining an amplitude from the received light intensity transmitted from the user device and calculating a concentration of particles in the subject from the amplitude.

22. A particle concentration measurement program that causes a computer of a device communicably connected to a user device including a radiator that radiates temporally periodic light to a subject and a received light intensity detection unit that is so disposed as to be separate from the radiator by a predetermined distance and detects a received light intensity of light emitted from the subject to carry out the process of determining an amplitude from the received light intensity transmitted from the user device and calculating a concentration of particles in the subject from the amplitude.

FIG. 1

(a)

(b)

FIG. 2

FIG. 3

LIGHT SOURCE

INCIDENT LIGHT

SIMULATED BLOOD VESSEL

FIG. 4

LIGHT REACHABLE
RANGE A

60 MINUTES
AFTER FAT IS LOADED

LIGHT REACHABLE
RANGE B

SIMULATED BLOOD VESSEL

240 MINUTES
AFTER FAT IS LOADED

INCIDENT POINT

FIG. 5

DEPTH-LUMINANCE GRAPH

LUMINANCE

400
300
200
100
0

0        200        400        600        800        1000        1200

ABOUT
1.5 mm

DEPTH

PHANTOM SURFACE        REACHABLE DEPTH

FIG. 6

FIG. 7

FIG. 8

FIG. 9

FIG. 10

FIG. 11

(a)

(b)

FIG. 12

241

CPU

24

ROM 243

RAM 244

HDD 245

EXTERNAL I/F 246

RECEIVED LIGHT INTENSITY DETECTION UNIT 22

INCIDENT LIGHT INTENSITY DETECTION UNIT 23

RADIATOR 25

242

FIG. 13

$y = 0.371x - 1.2452$
$R^2 = 0.86532$

CORRELATION COEFFICIENT

0.8
0.6
0.4
0.2
0
-0.2
-0.4
-0.6
-0.8
-1

0    1    2    3    4    5    6

BLOOD VESSEL DEPTH (mm)

FIG. 14

| CALCULATE FIRST AND SECOND RECEIVED LIGHT INTENSITIES | S107 |
| DETECT RECEIVED LIGHT INTENSITY | S101 |
| CALCULATE INCIDENT LIGHT INTENSITY | S105 |

CALCULATE FIRST AND SECOND RECEIVED LIGHT INTENSITIES — S107

CALCULATE FIRST AND SECOND TOTAL AMPLITUDES — S108

DETECT RECEIVED LIGHT INTENSITY — S101

CALCULATE TOTAL AMPLITUDE — S102

CALCULATE INCIDENT LIGHT INTENSITY — S105

CALCULATE TOTAL AMPLITUDE — S106

CALCULATE LIGHT PROPAGATION EFFICIENCY — S103

CALCULATE PARTICLE CONCENTRATION — S104

FIG. 15

RADIATION — S201

CALCULATE FIRST AND SECOND RECEIVED LIGHT INTENSITIES — S208

DETECT RECEIVED LIGHT INTENSITY — S202

CALCULATE INCIDENT LIGHT INTENSITY — S206

CALCULATE FIRST AND SECOND TOTAL AMPLITUDES — S209

CALCULATE TOTAL AMPLITUDE — S203

CALCULATE TOTAL AMPLITUDE — S207

CALCULATE LIGHT PROPAGATION EFFICIENCY — S204

CALCULATE PARTICLE CONCENTRATION — S205

## INTERNATIONAL SEARCH REPORT

| International application No. |
| --- |
| PCT/JP2019/040692 |

**A. CLASSIFICATION OF SUBJECT MATTER**
Int.Cl. A61B5/14557(2006.01)i, G01N21/17(2006.01)i

According to International Patent Classification (IPC) or to both national classification and IPC

**B. FIELDS SEARCHED**

Minimum documentation searched (classification system followed by classification symbols)
Int.Cl. A61B5/1455-5/1464, G01N21/00-21/01, G01N21/17-21/61, A61B10/00

| Documentation searched other than minimum documentation to the extent that such documents are included in the fields searched | |
| --- | --- |
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
JSTPlus/JMEDPlus/JST7580 (JDreamIII), PubMed

**C. DOCUMENTS CONSIDERED TO BE RELEVANT**

| Category* | Citation of document, with indication, where appropriate, of the relevant passages | Relevant to claim No. |
| --- | --- | --- |
| X <br><br> A | JP 2006-17746 A (HAMAMATSU PHOTONICS K.K.) 19 January 2006, paragraphs [0079]-[0087], [0111]-[0113], [0116], fig. 1-2, 4, 9-10 (Family: none) | 1, 5, 9, 13, 17-18, 20-2 2-4, 6-8, 10-12, 14-16, 19 |
| A | WO 2017/119130 A1 (MITSUBISHI CHEMICAL HOLDINGS CORP.) 13 July 2017, paragraphs [0012]-[0024], fig. 1-2 (Family: none) | 1-22 |

☒ Further documents are listed in the continuation of Box C.    ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 17 December 2019 (17.12.2019) | 07 January 2020 (07.01.2020) |

| Name and mailing address of the ISA/ <br> Japan Patent Office <br> 3-4-3, Kasumigaseki, Chiyoda-ku, <br> Tokyo 100-8915, Japan | Authorized officer <br><br><br> Telephone No. |
| --- | --- |

Form PCT/ISA/210 (second sheet) (January 2015)

**INTERNATIONAL SEARCH REPORT**

| International application No. |
| --- |
| PCT/JP2019/040692 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | WO 2017/141895 A1 (MEDICAL PHOTONICS CO., LTD.) 24 August 2017, paragraphs [0027]-[0029], [0036]-[0047], fig. 1 & JP 2017-144079 A & JP 5984074 B1 & US 2019/0046091 A1, paragraphs [0050]-[0052], [0059]-[0070], fig. 1 & EP 3417779 A1 & CN 108697388 A & KR 10-2018-0111956 A | 1-22 |
| A | JP 9-61359 A (HAMAMATSU PHOTONICS K.K.) 07 March 1997, paragraphs [0018]-[0050], fig. 1-4 & US 5772588 A, column 5, line 39 to column 10, line 7, fig. 1-4 & EP 0760477 A2 | 1-22 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014087825 A